# EUROPÄISCHE PATENTSCHRIFT

(11) **EP 3 509 510 B1**
(45) Veröffentlichungstag und Bekanntmachung des Hinweises auf die Patenterteilung: **05.08.2020**
(21) Anmeldenummer: 17761892.3
(22) Anmeldetag: 06.09.2017
(51) Int. Cl.: A61B 17/28, A61B 90/00

(54) **MEDIZINISCHES INSTRUMENT MIT REINIGUNGSSPALT IM VERSCHLUSSBEREICH**
MEDICAL INSTRUMENT WITH CLEANING GAP IN THE CLOSURE REGION
INSTRUMENT MÉDICAL PRÉSENTANT UNE FENTE DE NETTOYAGE DANS LA ZONE DE VERROUILLAGE

(30) Priorität: 06.09.2016 DE 102016116624
(43) Veröffentlichungstag der Anmeldung: 17.07.2019
(73) Patentinhaber: Karl Leibinger Medizintechnik GmbH & Co. KG, 78570 Mühlheim (DE)
(72) Erfinder: GABELE, Lorenz, 78570 Mühlheim (DE); ROMETSCH, Jürgen, 78570 Mühlheim (DE); SCHEU, Volker, 78570 Mühlheim (DE); SCHULZ, Oliver, 78570 Mühlheim (DE)
(74) Vertreter: Winter, Brandl, Fürniss, Hübner, Röss, Kaiser, Polte - Partnerschaft mbB
(86) Internationale Anmeldenummer: PCT/EP2017/072329
(87) Internationale Veröffentlichungsnummer: WO 2018/046534

(56) Entgegenhaltungen:
- WO-A1-2017/031506
- DE-U1-202009 014 310
- DE-U1-202013 010 321
- JP-A- H0 780 164
- JP-A- H04 224 785
- JP-U- S58 174 064

## Beschreibung

Die vorliegende Erfindung betrifft ein medizinisches Instrument bzw. Handwerkzeug, wie bspw. eine Klemme, eine Zange oder ein anderes scherenartiges Handwerkzeug, mit einem ersten Handhebel, der einen zum Betätigen per Hand vorgesehenen Handgriffabschnitt an seinem proximalen Ende, einen Arbeitsabschnitt, etwa nach Art einer Branche, zum Greifen, Klemmen oder Schneiden eines Objekts an seinem distalen Ende und einen dazwischenliegenden Lagerabschnitt aufweist, an dem ein Lagerelement angreift, um eine Schwenkbarkeit um eine Drehachse zwischen dem ersten Handhebel und einem zweiten Handhebel zu ermöglichen, wobei der zweite Handhebel einen Handgriffabschnitt an seinem proximalen Ende, einen Arbeitsabschnitt an seinem distalen Ende und einen dazwischenliegenden Führungsabschnitt aufweist, an dem das Lagerelement angreift, wobei der Führungsabschnitt des zweiten Handhebels auf einer Seite eine Einlegeausnehmung besitzt, um den Lagerabschnitt des ersten Handhebels so aufzunehmen, dass zwei Führungsüberstände distal und proximal der Einlegeausnehmung in einer Betriebsstellung, in der ein Greifen, Klemmen oder Schneiden stattfindet, den Lagerabschnitt wenigstens abschnittsweise überdecken.

Aus der DE 20 2013 010 321 U1 ist ein zangenartiges, medizinisches Instrument bekannt, welches aus einem ersten, eine Führungsplatte aufweisenden Handhebel und einem zweiten, eine Lagerplatte aufweisenden Handhebel besteht, welche über einen im Bereich der Führungsplatte und der Lagerplatte angeordneten und mit einem radial erweiterten Kopfteil versehenen Lagerzapfen schwenkbar miteinander verbunden sind, wobei die Lagerplatte in ihren längsseitigen Randbereichen jeweils eine Aufnahmenut bildet, in welche die Führungsplatte in ihrer Betriebsstellung eingreift, wobei die Lagerplatte zwischen den Aufnahmenuten eine sich über die komplette Breite der Lagerplatte erstreckende Vertiefung aufweist und die Führungsplatte aus ihrer Betriebsstellung in eine Reinigungsstellung relativ zur Lagerplatte drehbar ist, in welcher die Führungsplatte mit der Vertiefung in Überdeckung gelangt und im Bereich der Vertiefung zwischen der Führungsplatte und der Lagerplatte ein Distanzelement vorgesehen ist, dessen Höhe in Richtung des Lagerzapfens gleich groß oder kleiner ist als die Tiefe der Vertiefung.

Die DE 20 2010 010 843 U1 offenbart ein scherenartiges oder zangenartiges, medizinisches Handwerkzeug mit zwei jeweils zweiarmigen Handhebeln, die im Bereich zweier plattenartig abgeflachter Mittelabschnitte durch einen Gelenkzapfen gelenkig miteinander verbunden und gegeneinander verschwenkbar sind, wobei jeweils ein vorzugsweise kurzer Hebelarm jedes Handhebels als Werkzeug ausgebildet ist und der jeweils zweite, längere Hebelarm als Handgriff ausgebildet ist, wobei der im abgeflachten Mittelabschnitt des einen Handhebels unbeweglich befestigte Gelenkzapfen mit einem Ringflansch versehen ist, der von dem Mittelabschnitt des ersten Handhebels, in dem der Gelenkzapfen befestigt ist, einen Abstand aufweist, der mindestens der doppelten Dicke des auf dem Gelenkzapfen gelagerten Mittelabschnitts des zweiten Handhebels entspricht, sodass der zweite Handhebel in der maximalen Schwenklage der beiden Handhebel auf dem Gelenkzapfen wenigstens so weit in eine Lage verschiebbar ist, in welcher auch die einander gegenüberliegenden Innenflächen der abgeflachten Mittelabschnitte und der Gelenkzapfen freiliegen.

Die DE 20 2011 107 977 U1 wiederum offenbart ein scherenartiges oder zangenartiges, medizinisches Instrument mit einem ersten und einem zweiten Handhebel, welche jeweils ein Griffteil und ein Werkzeugteil aufweisen und welche über einen mit einem radial erweiterten Kopfteil versehenen Lagerzapfen schwenkbar miteinander in Verbindung stehen, wobei der erste Handhebel im Bereich zwischen seinem Griffteil und seinem Werkzeugteil eine Führungsplatte bildet und der zweite Handhebel im Bereich zwischen seinem Griffteil und seinem Werkzeugteil eine Lagerplatte bildet, in deren Bereich der Lagerzapfen feststehend angeordnet ist, wobei die Führungsplatte zur schwenkbaren Aufnahme des Lagerzapfens einen Durchbruch aufweist und wobei sich die Führungsplatte und die Lagerplatte im normalen Betriebszustand des Instruments flächig berühren, wobei der Durchbruch der Führungsplatte als ein sich in Längsrichtung der Führungsplatte erstreckendes Langloch ausgebildet ist und das Langloch im Randbereich seines dem Werkzeugteil benachbarten Endbereichs eine erste erweiterte Vertiefung und im Randbereich seines in Längsrichtung gegenüberliegenden Endbereichs eine zweite erweiterte Vertiefung aufweist, in welchen das Kopfteil des Lagerzapfens wahlweise aufnehmbar ist und die erste Vertiefung eine geringere Tiefe aufweist als die zweite Vertiefung und die Lagerplatte auf ihrer der Führungsplatte zugewandten Kontaktfläche eine im Bereich des Lagerzapfens angeordnete, quer zur Lagerplatte verlaufende Quernut aufweist, deren Tiefe zumindest der Tiefe der ersten Vertiefung des Langlochs entspricht und deren Breite zumindest der Breite der Führungsplatte entspricht und wobei der erste Handhebel mit seiner Führungsplatte in einer mit der Quernut fluchtende Schwenkposition bringbar und in Richtung des Lagerzapfens in die Quernut so weit verstellbar ist, dass das Kopfteil des Lagerzapfens mit der ersten Vertiefung des Langlochs außer Eingriff gelangt und das Kopfteil des Lagerzapfens durch eine Verstellung des zweiten Handhebels mit seiner Lagerplatte und dem Lagerzapfen entlang des Langlochs mit der zweiten Vertiefung des Langlochs in Überdeckung und durch eine Verstellung in Richtung des Lagerzapfens mit der zweiten Vertiefung in Eingriff bringbar ist und die Führungsplatte und die Lagerplatte in dieser Position einen Abstand voneinander aufweisen.

Benachbarter Stand der Technik ist auch aus den Druckschriften JP H04224785 A, JP H0780164 A und JP S58174064 U bekannt. Auch ist der Artikel von R. Ulrich "Über die Entwicklung zerlegbarer Klemmen- und Zangenschlüsse" aus "Die Medizinmechanik", vol. 58, 1937, Nr. 24, S. 239-242 bekannt.

Bei den aus dem Stand der Technik bekannten medizinischen Instrumenten sind die Führungsbereiche jedoch sehr minimal ausgebildet, sodass eine exakte und präzise Führung der beiden Handhebel zueinander nicht immer gewährleistet ist. Bei manchen der bekannten medizinischen Instrumente ist darüber hinaus die Montage bzw. Demontage des medizinischen Instruments mit größerem Aufwand verbunden.

Aufgabe der Erfindung ist es, die Nachteile aus dem Stand der Technik zu vermeiden oder wenigstens zu mildern, und insbesondere eine Beabstandung der beiden Handhebel in der Betriebsstellung umzusetzen, um ein Abfließen von Flüssigkeiten, wie bspw. Wundsekret, Blut, etc. bereits während der Nutzung zu ermöglichen, bzw. zu vereinfachen. Dies ermöglicht auch eine vereinfachte Reinigung und/oder Sterilisation des medizinischen Instruments.

Die Aufgabe der Erfindung wird bei einem gattungsgemäßen medizinischen Instrument dadurch gelöst, dass der Lagerabschnitt des ersten Handhebels durch ein für eine Beabstandung Kraft oder Widerstand zur Verfügung stellendes Abstandverursachungselement so in der Betriebsstellung von dem Führungsabschnitt des zweiten Handhebels weg gedrückt wird, dass sich ein Reinigungsspalt zwischen dem ersten Handhebel und dem zweiten Handhebel über die gesamte Länge des Führungsabschnitts erstreckt.

Vorteilhafte Ausführungsformen sind in den Unteransprüchen beansprucht und werden nachfolgend erläutert.

So ist es von Vorteil, wenn das Abstandverursachungselement als ein separates oder als ein integrales Bauteil ausgebildet ist. Ein separates Bauteil bietet den Vorteil, dass es einfach austauschbar ist, wohingegen ein integral ausgebildetes Bauteil die Gesamtkomponentenzahl reduziert und die Montage vereinfacht.

Dabei hat es sich als vorteilhaft herausgestellt, wenn das Abstandverursachungselement als ein Ring, als ein Bund oder als ein oder mehrere Steg / Stege ausgebildet ist.

Eine weitere vorteilhafte Ausführungsform sieht vor, dass das Abstandverursachungselement als eine Feder, ein Magnet oder ein Druckbehälter ausgebildet ist oder ein thermoaktives Element besitzt. Hierbei wird der Abstand zwischen dem Führungsabschnitt und dem Lagerabschnitt ausgebildet, indem das Abstandverursachungselement eine Kraft aufbringt, welche den Lagerabschnitt von dem Führungsabschnitt beabstandet.

Hierbei ist es insbesondere von Vorteil, wenn das Abstandverursachungselement derart ausgebildet ist, dass eine Reinigungsspalthöhe (gezielt) manuell einstellbar ist. Ein solches Abstandverursachungselement ermöglicht es, dem Benutzer des medizinischen Instruments die Reinigungsspalthöhe variabel individuell anpassen zu können.

Eine mögliche vorteilhafte Ausführungsform sieht vor, dass das Lagerelement als eine Schraube oder ein Niet ausgebildet ist, wobei eine Schraube im Gegensatz zu einem Niet einfacher (und zerstörungsfrei) demontierbar ist, wohingegen ein Niet sich nicht unbeabsichtigt lösen kann. Es sind jedoch auch andere form-, kraft- oder stoffschlüssige Verbindungen denkbar.

Ferner vorteilhaft ist es, wenn die Reinigungsspalthöhe in der Betriebsstellung kleiner ist als die Reinigungsspalthöhe in einer Reinigungsstellung. Durch eine vergrößerte Spalthöhe in der Reinigungsstellung wird somit die Reinigung und Sterilisation des medizinischen Instruments weiter vereinfacht, während in der Betriebsstellung durch die kleinere Reinigungsspalthöhe weiterhin eine exakte und präzise Führung der beiden Handhebel zueinander möglich ist.

Hierbei ist es von Vorteil, wenn die Reinigungsstellung einer Montagestellung der beiden Handhebel entspricht. So kann das medizinische Instrument zur Reinigung und/oder Sterilisation bei Bedarf auch demontiert werden.

Für das medizinische Instrument hat es sich als vorteilhaft herausgestellt, wenn die relative Verschwenkbarkeit der beiden Handhebel zueinander durch einen Rastmechanismus begrenzt ist, welcher vorzugsweise an dem proximalen Ende des jeweiligen Handgriffabschnitts angeordnet / vorgesehen ist. Somit kann verhindert werden, dass während einer Nutzung durch einen Bediener, wie bspw. ein Arzt, ein Operateur oder ein anderer Bediener, das medizinische Instrument versehentlich in die Reinigungsstellung gebracht wird.

Eine mögliche beispielhafte Ausführungsform sieht vor, dass der Arbeitsabschnitt des ersten Handhebels und der Arbeitsabschnitt des zweiten Handhebels gleich oder unterschiedlich ausgebildet sind. Dies ist abhängig davon, zu welchem Zweck das medizinische Instrument eingesetzt werden soll.

Ferner ist es von Vorteil, wenn die Handgriffabschnitte ergonomisch geformt sind. Unter einer ergonomischen Form wird hierbei verstanden, dass die Handgriffabschnitte eine solche Form aufweisen, dass sie ein Greifen, Halten und Bedienen des medizinisches Instruments mittels einer Hand eines Bedieners für diesen vereinfachen, bzw. angenehmer gestalten und dadurch ein leichteres und längeres Bedienen des medizinischen Instruments möglich ist, verglichen mit einer nicht ergonomischen Form, wie bspw., wenn die Handgriffabschnitte einfach nach Art einer Stange, ohne Krümmung und mit konstanter Dicke, ausgebildet sind. Ergonomische Formen können bspw. Krümmungen und/oder Vertiefungen umfassen, welche der Form/Kontur einer Hand und/oder Fingern nachempfunden sind, wenn diese das medizinische Instrument greifen/halten/bedienen.

Darüber hinaus hat es sich als vorteilhaft erwiesen, wenn in Längsrichtung verlaufende Außenkanten beider Führungsüberstände wenigstens ein Fünftel so lang sind wie die Länge der Einlegeausnehmung. Zusätzlich von Vorteil ist es hierbei, wenn die Länge der Einlegeausnehmung größer ist als die Breite des ersten Handhebels im Bereich des Lagerelements. Somit wird mithilfe der Führungsüberstände in der Betriebsstellung eine gute und präzise Führung des Lagerabschnitts ermöglicht, während gleichzeitig in der Reinigungs- bzw. Montagestellung eine einfache Reinigung bzw. Montage/Demontage erfolgen kann.

Eine andere beispielhafte Ausführungsform sieht vor, dass der Lagerabschnitt des ersten Handhebels die Führungsüberstände in einer Betriebsstellung gleitend berührt / kontaktiert und somit präzise geführt wird.

Hierbei hat es sich als vorteilhaft herausgestellt, wenn die Dicke des Lagerabschnitts kleiner ist als die Höhe eines zwischen einer Innenoberfläche der Führungsüberstände und der ihr zugewandten Oberfläche eines Verbindungsarms des Führungsabschnitts ausgebildeten Führungsspalts. Dadurch wird der Lagerabschnitt innerhalb dieses Führungsspalts präzise geführt und die damit einhergehende Relativbewegung der beiden Handhebel in der Betriebsstellung ist exakt ausführbar.

So sieht eine weitere vorteilhafte Ausführungsform bspw. vor, dass der Lagerabschnitt, etwa in der jeweiligen Mitte, eine geringere Dicke aufweist als der Handgriffabschnitt und/oder der Arbeitsabschnitt, etwa in deren jeweiliger Mitte. Dies macht insbesondere fertigungstechnisch gesehen Sinn, da so das Rohteil des medizinischen Instruments mit einer konstanten Dicke hergestellt werden kann und der Lagerabschnitt in einem anschließenden Schritt bspw. mittels Fräsen oder einem anderen materialabtragenden Verfahren auf die gewünschte Dicke gebracht werden kann, wobei optional (abhängig von dem gewählten Verfahren) gleichzeitig die für die Führung in den Führungsspalten notwendige Oberflächenbeschaffenheit erzeugt werden kann.

Weiter ist es von Vorteil, wenn die Schwenkbarkeit um die Drehachse einen relativen Verschwenkwinkel zwischen den Arbeitsabschnitten von 0° bis größer 90°, vorzugsweise bis 120°, zulässt. Dadurch ist es möglich, das medizinische Instrument auch in eine zur Reinigung und Sterilisation des medizinischen Instruments vorgesehene Reinigungsstellung zu bringen. Die Reinigungsstellung entspricht hierbei vorzugsweise einer Montagestellung, in der die beiden Handhebel mithilfe des Lagerelements verschwenkbar zueinander verbunden werden.

Darüber hinaus hat es sich als vorteilhaft herausgestellt, wenn der Lagerabschnitt und der Führungsabschnitt einander zugewandte Kontaktflächen aufweisen, die einander in der Betriebsstellung gleitend berühren / kontaktieren und somit eine präzise Führung der Handhebel zueinander realisiert wird.

Weiter ist es für das medizinische Instrument von Vorteil, wenn die Handhebel sowohl in die Betriebsstellung als auch in eine Reinigungsstellung bringbar sind. Die Reinigungsstellung entspricht hierbei vorteilhafterweise der Montagestellung, in der die zwei Handhebel mittels des Lagerelements miteinander verbunden werden.

Darüber hinaus ist es von Vorteil, wenn die Führungsüberstände zusammen mit der Kontaktfläche des Führungsabschnitts des zweiten Handhebels einen Hinterschnitt, bspw. in Form einer Führungsnut, ausbilden.

Weiter ist es von Vorteil, wenn die beiden Führungsüberstände jeweils eine Dicke besitzen, die zwischen ca. 80 % und ca. 120 % der Dicke des in der Einlegeausnehmung aufzunehmenden Lagerabschnitts entspricht, vorzugsweise (exakt) 100 %. Dadurch kann sichergestellt werden, dass die Führungsüberstände eine ausreichend hohe Stabilität aufweisen, um den Lagerabschnitt entsprechend präzise zu führen.

Eine andere vorteilhafte Ausführungsform sieht vor, dass die Führungsüberstände beidseitig der Einlegeausnehmung einander zugewandte Kanten / Ränder besitzen, die konvex oder ballig oder abgerundet ausgeformt sind. Dadurch werden scharfe Kanten, Ecken (z.B. durch einen überstehenden Grat) verhindert und somit das Verletzungsrisiko für den Bediener sowie für den Patienten reduziert.

Für die Führung des Lagerabschnitts ist es von Vorteil, wenn der proximale Führungsüberstand eine proximale Endkante besitzt, die parallel zu einer proximalen Endkante des Verbindungsarms und senkrecht zu der Längsachse des zweiten Handhebels ausgerichtet ist.

Aus den gleichen Gründen ist es von Vorteil, wenn der distale Führungsüberstand eine distale Endkante besitzt, die parallel zu einer distalen Endkante des Verbindungsarms und senkrecht zu der Längsachse des zweiten Handhebels ausgerichtet ist.

Hierbei hat es sich als vorteilhaft herausgestellt, wenn der Flächenträgheitsschwerpunkt des Führungsüberstands auf der in Längsrichtung verlaufenden Symmetrieachse liegt. Dadurch ist eine gute Bedienbarkeit des medizinischen Instruments möglich.

Eine andere denkbare Ausführungsform betrifft ein medizinisches Instrument, mit einem ersten Handhebel, der einen zum Betätigen per Hand vorgesehenen Handgriffabschnitt an seinem proximalen Ende, einen Arbeitsabschnitt zum Greifen, Klemmen oder Schneiden eines Objekts an seinem distalen Ende und einen dazwischenliegenden Lagerabschnitt aufweist, an dem ein Lagerelement angreift, um eine Schwenkbarkeit um eine Drehachse zwischen dem ersten Handhebel und einem zweiten Handhebel zu ermöglichen, wobei der zweite Handhebel einen Handgriffabschnitt an seinem proximalen Ende, einen Arbeitsabschnitt an seinem distalen Ende und einen dazwischenliegenden Führungsabschnitt aufweist, an dem das Lagerelement angreift, wobei eine Lagerung derart ausgebildet ist, dass die beiden Handhebel in einer Betriebsstellung mittels des Lagerelements formschlüssig, jedoch zueinander verschwenkbar gelagert sind, und dass die Handhebel in einer Reinigungsstellung werkzeuglos demontierbar sind.

Eine weitere vorteilhafte Ausführungsform sieht vor, dass eine Lagerung der zwei Handhebel zueinander nach Art eines Bajonettverschlusses ausgebildet ist. Dadurch ist sowohl eine werkzeuglose Montage wie auch Demontage möglich. Der Bereich, in dem der Bajonettverschluss geschlossen ist, d.h., die beiden Handhebel miteinander in Formschluss sind, entspricht hierbei der Betriebsstellung.

Hierfür hat es sich als vorteilhaft erwiesen, wenn in dem Lagerabschnitt des ersten Handhebels das Lagerelement integral vorgesehen ist. Das vereinfacht zum einen die Montage und reduziert zum anderen die Gesamtkomponentenzahl.

Ferner ist es von Vorteil, wenn das Lagerelement im Wesentlichen rund, d.h. in der Schwenkebene (die Ebene senkrecht zur Drehachse) kreisförmig ausgebildet ist und in Umfangsrichtung vorzugsweise gleichverteilte, bzw. 2, 3, 4, 5 oder 6 Vorsprünge / Überstände aufweist. Diese dienen der formschlüssigen Verbindung nach Art eines Bajonettverschlusses des ersten Handhebels mit dem zweiten Handhebel.

Hierfür ist es von Vorteil, wenn der Führungsabschnitt des zweiten Handhebels im Wesentlichen rund, d.h. in der Schwenkebene (die Ebene senkrecht zur Drehachse) kreisförmig ausgebildet ist und einen in Umfangsrichtung verlaufenden Führungsspalt aufweist. In diesen greifen die Vorsprünge des in den ersten Handhebel integrierten Lagerelements ein bzw. wirken mit diesen zusammen und bilden somit eine bajonettartige Verschlussart aus.

Eine weitere vorteilhafte Ausführungsform sieht vor, dass der umlaufende Führungsspalt durch mehrere in Umfangsrichtung vorzugsweise gleichverteilte Ausnehmungen unterbrochen ist. In diese können die Vorsprünge des Lagerelements eingesetzt werden, d.h., das medizinische Instrument befindet sich in der Reinigungs- bzw. Montage-/Demontagestellung, wenn die Vorsprünge in den Ausnehmungen positioniert sind.

Hierbei ist es von Vorteil, wenn die Anzahl und die Position entlang des Umfangs der jeweiligen Vorsprünge / Überstände der Anzahl und der Position entlang des Umfangs der jeweiligen Ausnehmungen entspricht. Nur so ist eine einfache Montage bzw. Demontage des medizinischen Instruments möglich.

Eine weitere mögliche Ausführungsform sieht vor, dass das Lagerelement in Form eines Zapfens ausgebildet ist, der sich entlang der Drehachse erstreckt, wobei der Führungsabschnitt des zweiten Handhebels auf einer Seite die Einlegeausnehmung besitzt, um den Lagerabschnitt des ersten Handhebels so aufzunehmen, dass die zwei Führungsüberstände distal und proximal der Einlegeausnehmung in der Betriebsstellung, in der ein Greifen, Klemmen oder Schneiden stattfindet, den Lagerabschnitt wenigstens abschnittsweise überdecken. Zur Realisierung der schwenkbaren Verbindung des ersten Handhebels mit dem zweiten Handhebel hat es sich als vorteilhaft herausgestellt, wenn der Lagerabschnitt des ersten Handhebels ein Loch aufweist, in welches der Zapfen einsteckbar / durchsteckbar ist.

Vorteilhafte Ausführungsformen der Vorsprünge / Überstände umfassen bspw., dass diese trapezformartig oder zapfenartig ausgebildet sind. Jedoch sind auch jegliche andere geometrische Formen denkbar.

Ferner vorteilhaft ist es, wenn die relative Verschwenkung der beiden Handhebel zueinander in der Betriebsstellung durch die Anzahl und Positionierung der über den Umfang verteilten Ausnehmungen definiert ist.

Mit anderen Worten besteht die Erfindung darin, dass ein, insbesondere zur Reinigung und/oder Sterilisation des medizinischen Instruments vorgesehener Reinigungsspalt zwischen dem ersten Handhebel und dem zweiten Handhebel bereits in der Betriebsstellung ausgebildet ist und sich über die gesamte Länge des Führungsabschnitts erstreckt.

Die Erfindung wird nachfolgend mit Hilfe von Figuren näher erläutert, in denen unterschiedliche Ausführungsformen dargestellt sind. Es zeigen:
- Fig. 1: eine perspektivische Ansicht eines medizinischen Instruments einer ersten beispielhaften Ausführungsform in einer Montage- bzw. Reinigungsstellung;
- Fig. 2: eine perspektivische Ansicht des medizinischen Instruments der ersten beispielhaften Ausführungsform in einer Arbeits- bzw. Betriebsstellung;
- Fig. 3: eine Draufsicht einer zweiten beispielhaften Ausführungsform des medizinischen Instruments in der Montage- bzw. Reinigungsstellung;
- Fig. 4: eine Ansicht von oben der zweiten beispielhaften Ausführungsform, in Richtung des Pfeils IV aus Fig. 3 gesehen;
- Fig. 5: eine perspektivische Ansicht eines zweiten Handhebels des medizinischen Instruments einer dritten beispielhaften Ausführungsform;
- Fig. 6: eine perspektivische Ansicht eines ersten Handhebels des medizinischen Instruments der dritten Ausführungsform;
- Fig. 7: eine perspektivische Ansicht des ersten Handhebels und des zweiten Handhebels des medizinischen Instruments in der dritten beispielhaften Ausführungsform in einer montierten Position;
- Fig. 8: eine perspektivische Draufsicht des medizinischen Instruments in einer vierten beispielhaften Ausführungsform in der Montage- bzw. Reinigungsstellung;
- Fig. 9: eine Draufsicht, entlang des Pfeils IX aus Fig. 8 gesehen, des medizinischen Instruments in der vierten beispielhaften Ausführungsform;
- Fig. 10: eine Draufsicht des medizinischen Instruments in einer fünften beispielhaften Ausführungsform;
- Fig. 11: eine Seitenansicht des medizinischen Instruments der fünften beispielhaften Ausführungsform;
- Fig. 12: eine ausschnittsweise, vergrößert dargestellte Ansicht des medizinischen Instruments der fünften Ausführungsform im Bereich der Verbindungsstelle des ersten Handhebels und des zweiten Handhebels in einer ersten Position;
- Fig. 13: eine Detailansicht des Lagerelements in einer zweiten möglichen Position;
- Fig. 14: eine perspektivische Ansicht des medizinischen Instruments in einer sechsten beispielhaften Ausführungsform in einem demontierten Zustand; und
- Fig. 15: eine perspektivische Ansicht des medizinischen Instruments der sechsten beispielhaften Ausführungsform in der Betriebsstellung.

Die Figuren sind lediglich schematischer Natur und dienen nur dem Verständnis der Erfindung. Die gleichen Elemente sind mit denselben Bezugszeichen versehen.

Merkmale der einzelnen Ausführungsbeispiele können auch in anderen Ausführungsbeispielen realisiert werden. Sie sind also untereinander austauschbar.

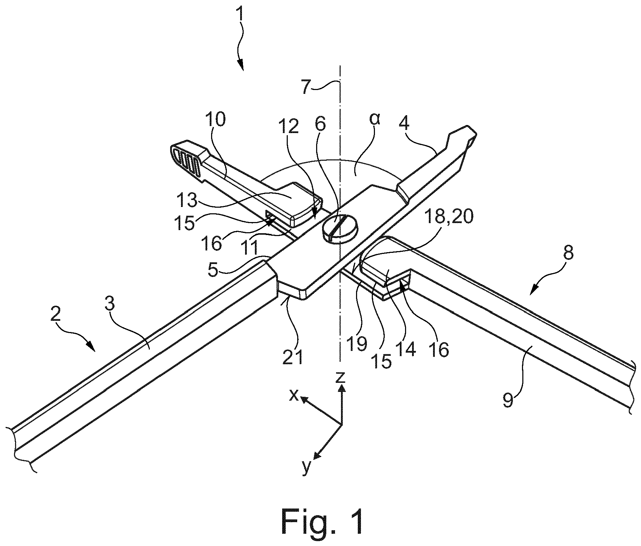

Fig. 1 zeigt ein medizinisches Instrument 1 einer ersten beispielhaften Ausführungsform in einer perspektivischen Ansicht, das einen ersten Handhebel 2 umfasst, welcher im Wesentlichen aus drei Abschnitten aufgebaut ist: einem Handgriffabschnitt 3, einem Arbeitsabschnitt 4 und einem Lagerabschnitt 5.

Der Handgriffabschnitt 3 befindet sich an einem proximalen Ende des ersten Handhebels 2 und dient einem Betätigen per Hand durch einen Nutzer, wie bspw. einem Chirurgen bzw. Operateur oder Arzt. Der Arbeitsabschnitt 4 ist etwa nach Art einer Branche ausgebildet, und dient dem Greifen, Klemmen oder Schneiden eines Objekts, wie bspw. Gewebe eines Patienten, und befindet sich an einem distalen Ende des ersten Handhebels 2. Zwischen dem Handgriffabschnitt 3 und dem Arbeitsabschnitt 4 befindet sich der Lagerabschnitt 5, welcher im Wesentlichen dazu dient, den ersten Handhebel 2 mittels eines Lagerelements 6 mit einem zweiten Handhebel 8 um eine gemeinsame Drehachse 7 verschwenkbar zu verbinden.

Der zweite Handhebel 8 umfasst einen Handgriffabschnitt 9, einen Arbeitsabschnitt 10 und einen Führungsabschnitt 11. Der Handgriffabschnitt 9 befindet sich analog zum Handgriffabschnitt 3 des ersten Handhebels 2, an einem proximalen Ende des zweiten Handhebels 8 und der Arbeitsabschnitt 10 befindet sich an einem distalen Ende des zweiten Handhebels 8. Zwischen dem Handgriffabschnitt 9 und dem Arbeitsabschnitt 10 ist der Führungsabschnitt 11 angeordnet.

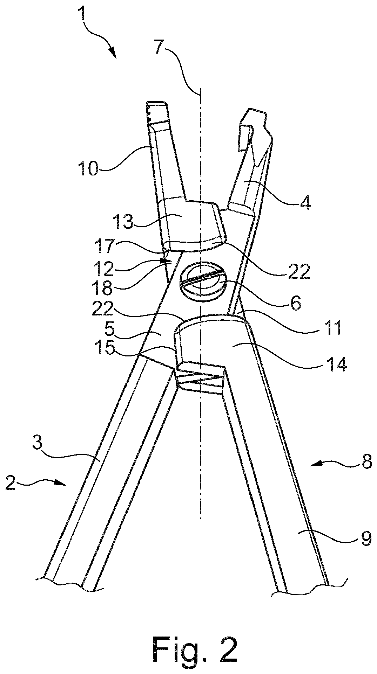

Der Führungsabschnitt 11 besitzt auf einer Seite eine Einlegeausnehmung 12, welche es ermöglicht, dass der Lagerabschnitt 5 in einer Reinigungs- bzw. Montagestellung (wie in Fig. 1 gezeigt) einfach montierbar ist und in einer Arbeits- bzw. Betriebsstellung (siehe Fig. 2) mittels zweier Führungsüberstände 13, 14, welche sich distal und proximal der Einlegeausnehmung 12 befinden, den Lagerabschnitt 5 wenigstens abschnittsweise überdecken und somit den ersten Handhebel 2 gegenüber dem zweiten Handhebel 8 führen.

Die Montage- bzw. Reinigungsstellung entspricht einer solchen Stellung, in der ein relativer Verschwenkwinkel a, zwischen den zwei Arbeitsabschnitten 4, 10 gemessen, so gewählt ist, dass der Lagerabschnitt 5 nicht mit den Führungsüberständen 13, 14 in Eingriff ist, bzw. von diesen nicht überdeckt wird. Dies entspricht einer solchen relativen Stellung der beiden Handhebel 2, 8 zueinander, dass der erste Handhebel 2 im Bereich des Lagerabschnitts 5 mit Hilfe des Lagerelements 6 einfach mit dem zweiten Handhebel 8 verbunden bzw. daran montiert werden kann.

Eine Arbeits- bzw. Betriebsstellung des medizinischen Instruments 1, wie in Fig. 2 gezeigt, beschreibt eine Stellung bzw. sämtliche Stellungen in einem vorgegebenen Winkel (innerhalb eines Winkelbereichs), in dem der erste Handhebel 2 mittels des Lagerabschnitts 5 durch die Führungsüberstände 13, 14 relativ zum zweiten Handhebel 8 geführt wird. In der Betriebsstellung findet ein Greifen, Klemmen oder Schneiden eines Objekts, wie bspw. menschliches Gewebe, statt.

Die relative Verschwenkbarkeit der beiden Handhebel 2, 8 kann mittels eines Rastmechanismus (hier nicht gezeigt) begrenzt sein, welcher bspw. an den proximalen Enden des jeweiligen Handgriffabschnitts 3, 9 vorgesehen ist. Dadurch kann sichergestellt werden, dass der Bediener das medizinische Instrument 1 während der Bedienung / Nutzung nicht unbeabsichtigt in die Montage- und Reinigungsstellung bringen kann.

Die Führungsüberstände 13, 14 weisen beidseitig in Längsrichtung des zweiten Handhebels 8 verlaufende Außenkanten 15 auf, die wenigstens ein Fünftel, vorzugsweise ein Viertel, so lang sind wie eine Länge der Einlegeausnehmung 12, wodurch eine sichere Führung des Lagerabschnitts 5 über den gesamten Betriebsbereich möglich ist.

Die Länge der Einlegeausnehmung 12 ist hierbei größer als die Breite des ersten Handhebels 2 im Bereich des Lagerelements 6. Dadurch ist gewährleistet, dass eine einfache Montage bzw. Demontage des medizinischen Instruments 1 möglich ist.

Die Länge der Einlegeausnehmung 12 wird hierbei in Längsrichtung des zweiten Handhebels 8 gesehen und entspricht der X-Richtung des Koordinatensystems aus Fig. 1. Die Längsrichtung des ersten Handhebels 2 entspricht in Fig. 1 der Y-Richtung und die Breite des ersten Handhebels 2 erstreckt sich in der XY-Ebene, welches der Schwenkebene des medizinischen Instruments 1 entspricht, entlang der X-Richtung (senkrecht zur Y-Richtung). Eine Dicke jeglicher Abschnitte des ersten Handhebels 2 und des zweiten Handhebels 8 erstreckt sich in Z-Richtung des gezeigten Koordinatensystems.

Sowohl aus Fig. 1, insbesondere jedoch aus Fig. 2, ist gut erkennbar zu entnehmen, dass die Dicke des Lagerabschnitts 5 kleiner ist als die Höhe eines Führungsspalts 16, welcher zwischen einer Innenoberfläche 17 der Führungsüberstände 13, 14 und einer ihrer zugewandten Oberfläche eines so genannten Verbindungsarms 19 des Führungsabschnitts 11 ausgebildet ist. Die Oberfläche ist eine Innenoberfläche 18 des Verbindungsarms 19 und dient insbesondere in der Betriebsstellung als Führungs-und Kontaktoberfläche 20, die einer Kontaktoberfläche 21 (untere Oberfläche in Fig. 1) des Lagerabschnitts 5 zugewandt ist, wobei diese beiden Kontaktoberflächen 20, 21 in der Betriebsstellung einander gleitend berühren.

Die Führungsüberstände 13, 14 weisen jeweils eine der Einlegeausnehmung 12 zugewandte Kante 22 (siehe Fig. 2) auf, die vorzugsweise, wie u.a. in Fig. 1 und Fig. 2 gezeigt, konvex, ballig oder abgerundet ausgeformt ist.

Die Arbeitsabschnitte 4, 10 können, wie in Fig. 1 und Fig. 2 gezeigt, unterschiedlich ausgebildet sein, können jedoch alternativ auch gleich ausgebildet sein. Die Arbeitsabschnitte 4, 10, wie hier gezeigt, sind lediglich beispielhaft zu sehen und es sind auch andere Ausprägungen der Arbeitsabschnitte 4, 10 denkbar.

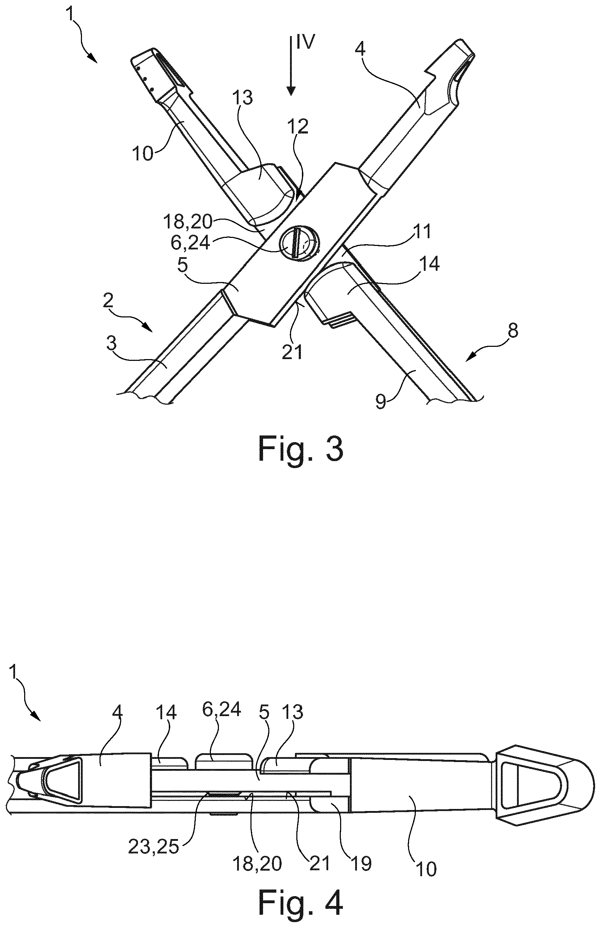

Fig. 3 und Fig. 4 zeigen eine zweite beispielhafte Ausführungsform des medizinischen Instruments 1. Diese zweite beispielhafte Ausführungsform sieht vor, dass sich die Kontaktoberflächen 20, 21 (siehe Fig. 4) nun nicht mehr berühren, da zwischen ihnen ein Abstandverursachungselement 23 positioniert ist, welches die Kontaktoberflächen 20, 21 zueinander beabstandet. Fig. 3 zeigt das medizinische Instrument 1 der zweiten beispielhaften Ausführungsform in der Montage- und Reinigungsstellung, wobei Fig. 3 im Wesentlichen Fig. 1 entspricht. Der Unterschied zwischen der in Fig. 1 gezeigten ersten beispielhaften Ausführungsform und der in Fig. 3 gezeigten zweiten beispielhaften Ausführungsform wird erst durch die in Fig. 4 gezeigte Ansicht ersichtlich.

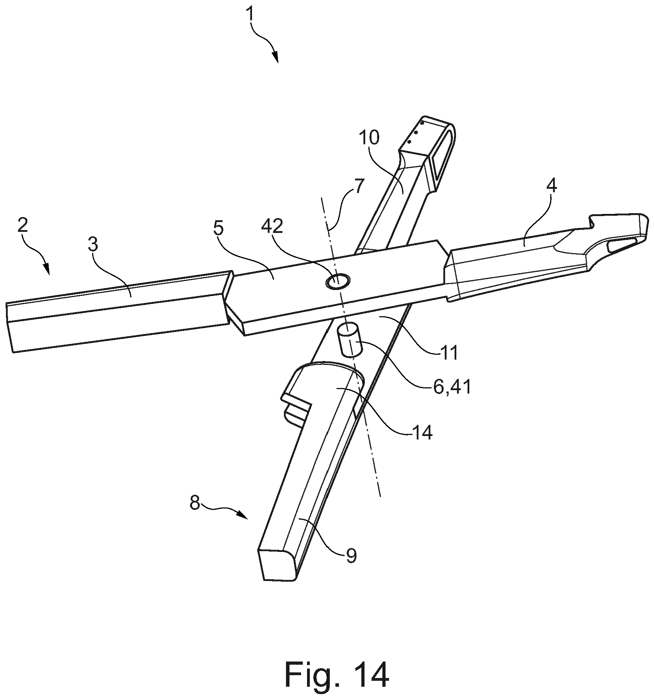

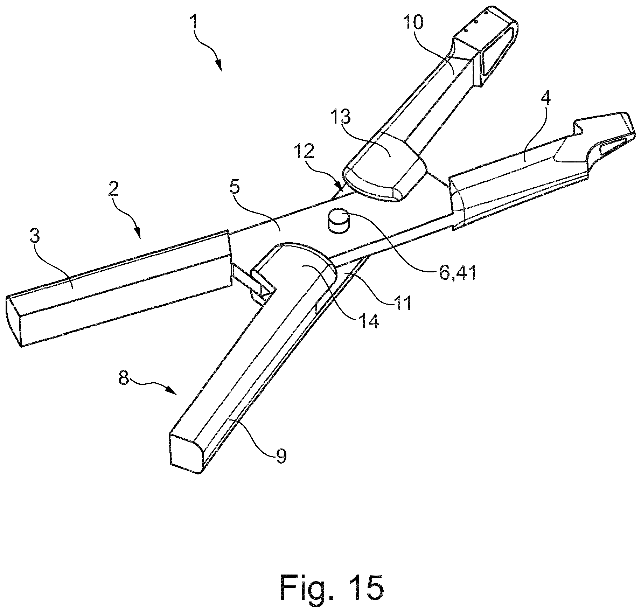

In der zweiten beispielhaften Ausführungsform, wie auch schon in der ersten gezeigten beispielhaften Ausführungsform ist das Lagerelement 6 als eine Schraube 24 ausgebildet. Alternative Ausführungsformen des Lagerelements 6 sehen bspw. auch ein Niet vor. Es sind jedoch auch weitere Verbindungselemente als Lagerelement 6, wie bspw. ein Zapfen (siehe hierzu auch Fig. 14 und Fig. 15), denkbar.

In Fig. 4 ist das Abstandverursachungselement 23 als ein widerstandbildendes Element in Form eines separaten Rings 25 ausgebildet. Alternativ kann das Abstandverursachungselement 23 auch als ein integral ausgebildeter Ring, bspw. in Form eines umlaufenden Bundes oder als beidseitig des Lagerelements 6 ausgebildete Stege ausgebildet sein. Ein Vorteil des separaten Bauteils ist u.a. die leichte Austauschbarkeit des Abstandverursachungselements 23, welche es ermöglicht, mithilfe der Verwendung verschiedener Abstandverursachungselemente den Abstand zwischen dem Führungsabschnitt 11 und dem Lagerabschnitt 5 zu variieren.

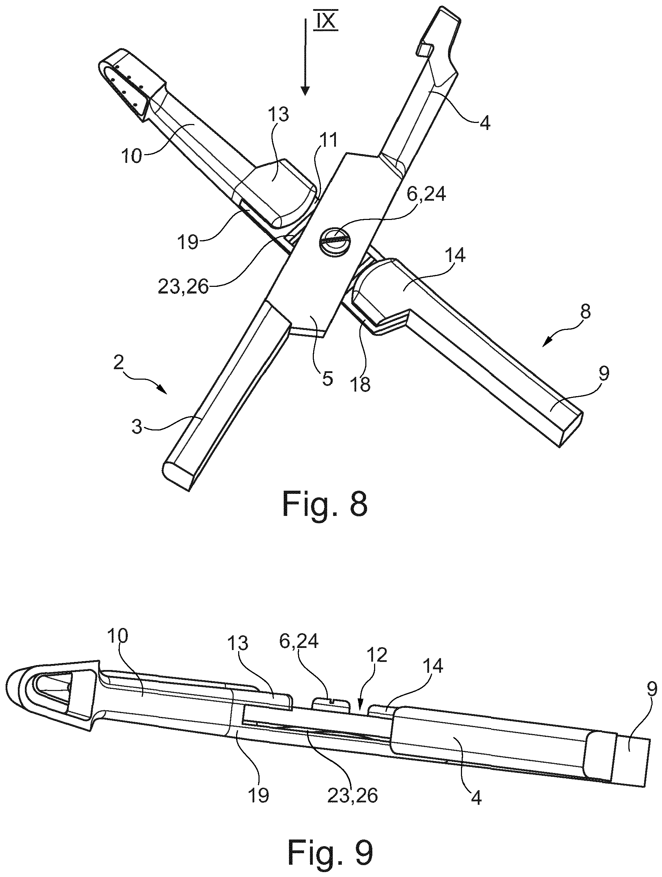

Statt einen Widerstand darzustellen, kann das Abstandverursachungselement 23 auch, wie beispielhaft in einer vierten Ausführungsform (siehe Fig. 8 und Fig. 9) gezeigt, als ein kraftaufbringendes Element, bspw. in Form einer Blattfeder 26 ausgebildet sein. Zu solchen kraftaufbringenden Elementen zählen u.a. auch andere Arten von Federn, wie bspw. eine Tellerfeder oder eine Spiralfeder, und darüber hinaus auch Magnete, wobei hier die magnetische Abstoßung zur Beabstandung des Lagerabschnitts 5 und der Innenoberfläche 18 des Führungsabschnitts 11 genutzt wird, oder alternativ ist auch ein Druckbehälter möglich. Ferner ist auch ein thermoaktives Element denkbar, welches sich bspw. ab einer bestimmten Temperatur ausdehnt und so zur Beabstandung führt.

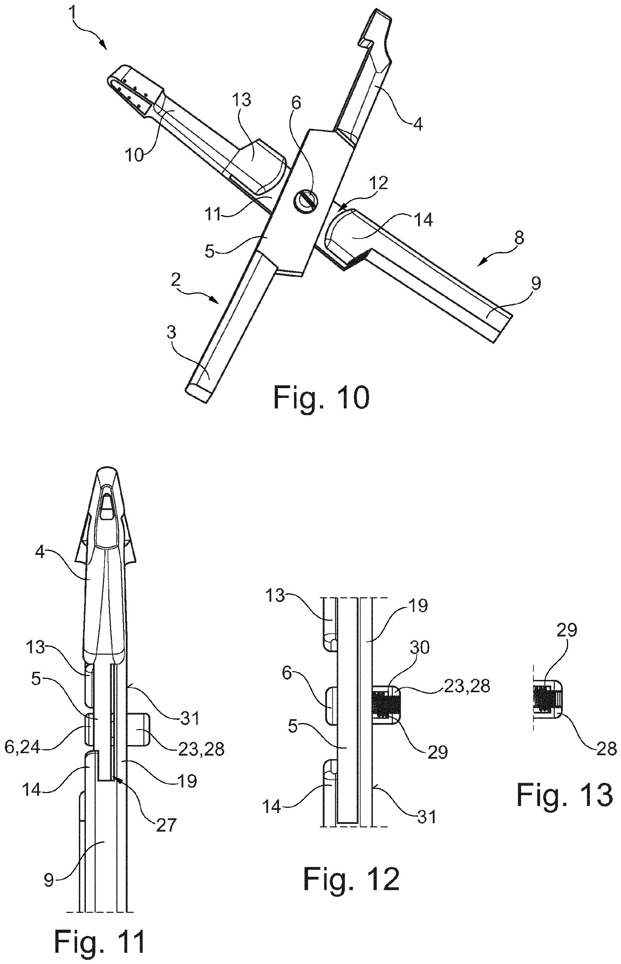

Die Fign. 10 bis 13 zeigen eine fünfte beispielhafte Ausführungsform des medizinischen Instruments 1, in dem ein Abstandverursachungselement 23 vorgesehen ist, welches eine gezielte manuelle Einstellbarkeit der Höhe (entlang/in Richtung der Drehachse 7 gesehen) des Reinigungsspalts 27 ermöglicht. Hierbei zeigt Fig. 10 eine Draufsicht auf die fünfte beispielhafte Ausführungsform des medizinischen Instruments 1 in der Montage- und Reinigungsstellung, welche im Wesentlichen der in Fig. 1 und Fig. 3 gezeigten Darstellungen entspricht.

Das in dieser Ausführungsform vorgesehene Abstandverursachungselement 23 ist derart ausgebildet, dass an dem Verbindungsarm 19 (siehe Fig. 11) eine Abstandshülse 28 befestigt ist, in welche das Lagerelement 6, welches als Schraube 24 ausgebildet ist, eingeschraubt werden kann. Das heißt, die Abstandshülse 28 weist in ihrem Inneren ein Innengewinde auf, in das die Schraube 24 eingeschraubt / gedreht werden kann.

Wie aus Fig. 12 und Fig. 13 ersichtlich, befindet sich in der Abstandshülse 28 eine Spiralfeder 29, welche in einem innerhalb der Abstandshülse 28 ausgebildeten Topf 30 angeordnet ist, wobei der Topf 30 einen größeren Innendurchmesser aufweist als der Außendurchmesser des Innengewindes. Die Spiralfeder 29 liegt mit ihrem einen Ende am Grund/Boden des Topfs 30 auf und mit dem anderen Ende an der Außenseite 31 des Verbindungsarms 19 an. Über die Einschraubtiefe des Lagerelements 6 in das Innengewinde der Abstandshülse 28 und der durch die Vorspannung der Spiralfeder 29 entstehende Kraft, die auf die Außenseite 31 des Verbindungsarms 19 wirkt, kann die Höhe des Reinigungsspalts 27 gezielt eingestellt werden.

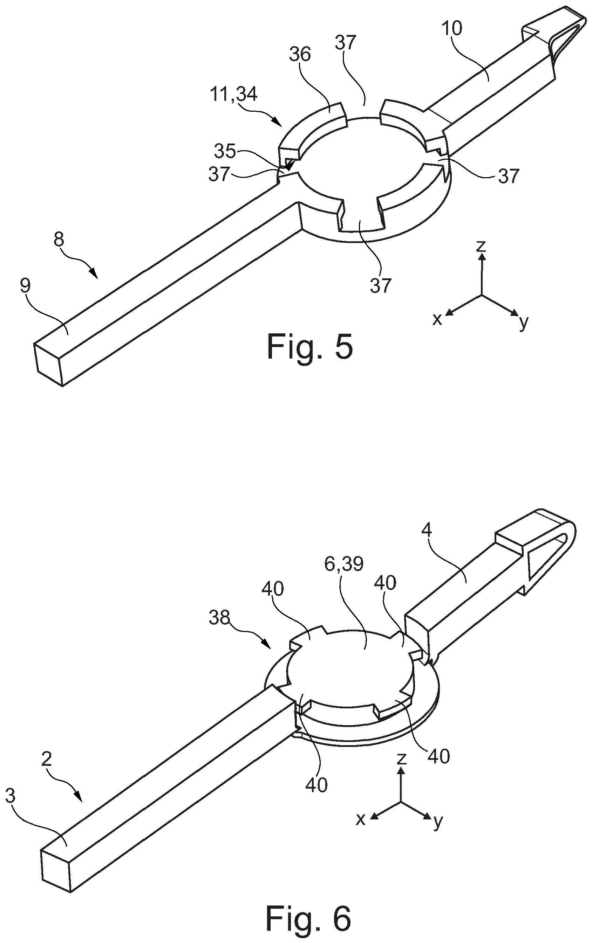

Die Fign. 5 bis 7 sowie die Fign. 14 und 15 zeigen eine dritte bzw. sechste beispielhafte Ausführungsform, welche jeweils eine werkzeuglos demontierbare Verbindung der Handhebel 2, 8 vorsieht.

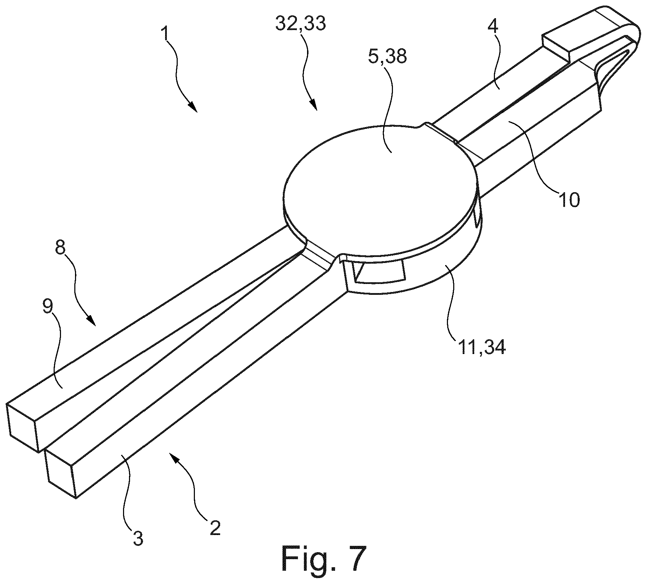

Die in Fig. 5 bis. Fig. 7 gezeigte dritte beispielhafte Ausführungsform stellt hierbei eine Lagerung 32 nach Art eines Bajonettverschlusses 33 (siehe Fig. 7) dar. Hierfür weist bspw. der zweite Handhebel 8 als Führungsabschnitt 11 einen in der XY-Ebene im Wesentlichen kreisförmig / rund ausgebildeten Führungsabschnitt 34 auf. Der Führungsabschnitt 34 weist einen in Umfangsrichtung verlaufenden Führungsspalt 35 auf, der durch einen in Umfangsrichtung verlaufenden Führungsüberstand 36 ausgebildet wird, wobei sich der Führungsüberstand 36 von einem Außenumfang des Führungsabschnitts 34 radial nach innen erstreckt.

Der umlaufende Führungsspalt 35 bzw. der umlaufende Führungsüberstand 36 werden durch mindestens einen, vorzugsweise mehrere (in Fig. 5, vier), Ausnehmungen 37 unterbrochen, welche vorzugsweise entlang der Umfangsrichtung gleichverteilt angeordnet sind.

Der in Fig. 6 gezeigte erste Handhebel 2 umfasst den Handgriffabschnitt 3 und den Arbeitsabschnitt 4, wie bereits aus den anderen Ausführungsformen schon bekannt. Dazwischenliegend befindet sich der Lagerabschnitt 5 in Form eines in der XY-Ebene aus Fig. 6 im Wesentlichen kreisförmig bzw. rund ausgebildeten Lagerabschnitts 38, der das Gegenstück zum in Fig. 5 gezeigten Führungsabschnitt 34 ausbildet.

In dem Lagerabschnitt 38 ist das Lagerelement 6 integral vorgesehen und wird nachfolgend auch als Lagerelement 39 bezeichnet. Das Lagerelement 39 ist wie der Lagerabschnitt 38 in der XY-Ebene im Wesentlichen kreisförmig ausgebildet, erstreckt sich entlang einer Höhenrichtung (Z-Richtung in Fig. 6) und weist einen kleineren Außendurchmesser auf als der Lagerabschnitt 38. Dieses kreisförmige Lagerelement 39 weist mindestens einen, vorzugsweise mehrere bzw. 2, 3, 4, 5 oder 6 (in Fig. 6, vier) Vorsprünge bzw. Überstände 40 auf, die sich von einem Außenumfang des Lagerelements 39 radial nach außen erstrecken.

Die Anzahl der vorhandenen Vorsprünge 40 und ihre Positionierung entlang des Umfangs entspricht der Anzahl und Positionierung der jeweiligen Ausnehmungen 37 am Führungsabschnitt 34. Der Führungsabschnitt 34 und der Lagerabschnitt 38 sind derart aufeinander abgestimmt, dass sie formschlüssig ineinandergreifen und durch ein relatives Verdrehen der beiden Handhebel 2, 8 ein Bajonettverschluss 33 (siehe Fig. 7) realisiert wird.

Die Betriebsstellung erstreckt sich dabei über den Bereich, der durch die Führungsvorsprünge 36 definiert ist. Das heißt, der Verstellwinkel α ist abhängig von der Anzahl und Positionierung der Führungsüberstände 36 bzw. der Ausnehmungen 37. In dem in Fig. 5 bis Fig. 7 gezeigten Ausführungsbeispiel ist der Verstellwinkel α bspw. kleiner 90°. Jedoch kann durch eine nicht gleichmäßige Verteilung der Vorsprünge 40 (und dadurch auch eine nicht gleichmäßige Verteilung der Ausnehmungen 37) ein größerer Verstellwinkel α (z.B., größer als 90°) realisiert werden. Alternativ ist es auch denkbar, die Vorsprünge 40 verschiedenartig auszubilden, so dass nicht jeder Vorsprung in jede Ausnehmung 37 passt.

Die in dieser Ausführungsform gezeigten Vorsprünge 40 sind trapezformartig ausgebildet. Alternativ sind auch andere geometrische Formen, wie bspw. ein Zapfen oder eine Dreiecksform etc. denkbar, die im Zusammenspiel mit den dazu passend ausgebildeten Ausnehmungen 37 am Führungsabschnitt 34, ein bajonettverschlussartiges Verbinden der beiden Handhebel 2, 8 ermöglichen.

Fig. 14 und Fig. 15 zeigen eine sechste beispielhafte Ausführungsform, welche im Wesentlichen der in Fig. 1 und Fig. 2 gezeigten ersten Ausführungsform entspricht, mit dem Unterschied, dass als Lagerelement 6 lediglich ein Zapfen 41 vorgesehen ist. Dieser ist hier integral mit dem zweiten Handhebel 8 ausgebildet und wird bei der Montage des medizinischen Instruments 1 in der Montage- und Reinigungsstellung durch ein Loch 42 durchgesteckt bzw. eingesteckt, welches sich in dem Lagerabschnitt 5 des ersten Handhebels 2 befindet. In der Betriebsstellung werden die beiden Handhebel 2, 8 mittels der Führungsüberstände 13, 14 (siehe Fig. 15) relativ zueinander geführt. In der Montage- und Reinigungsstellung (siehe Fig. 14) können die beiden Handhebel 2, 8 werkzeuglos voneinander getrennt werden, was einer werkzeuglosen Demontage des medizinischen Instruments 1, bspw. zur Reinigung und Sterilisation, entspricht.

Alternativ kann das Lagerelement 6 auch als ein separat ausgebildeter Zapfen vorgesehen sein, bspw. in Form eines Gewindezapfens, welcher dann in ein an dem Führungsabschnitt 11 vorgesehenes Gewinde eingeschraubt werden kann.

### Bezugszeichenliste

- 1: medizinisches Instrument
- 2: erster Handhebel
- 3: Handgriffabschnitt
- 4: Arbeitsabschnitt
- 5: Lagerabschnitt
- 6: Lagerelement
- 7: Drehachse
- 8: zweiter Handhebel
- 9: Handgriffabschnitt
- 10: Arbeitsabschnitt
- 11: Führungsabschnitt
- 12: Einlegeausnehmung
- 13: Führungsüberstand
- 14: Führungsüberstand
- 15: Außenkante
- 16: Führungsspalt
- 17: Innenoberfläche
- 18: Innenoberfläche
- 19: Verbindungsarm
- 20: Führungs- und Kontaktoberfläche
- 21: Kontaktoberfläche
- 22: Kante
- 23: Abstandverursachungselement
- 24: Schraube
- 25: Ring
- 26: Blattfeder
- 27: Reinigungsspalt
- 28: Abstandshülse
- 29: Spiralfeder
- 30: Topf
- 31: Außenseite
- 32: Lagerung
- 33: Bajonettverschluss
- 34: Führungsabschnitt
- 35: Führungsspalt
- 36: Führungsüberstand
- 37: Ausnehmung
- 38: Lagerabschnitt
- 39: kreisförmiges Lagerelement
- 40: Vorsprünge
- 41: Zapfen
- 42: Loch

- α: Verstellwinkel

## Patentansprüche

1. Medizinisches Instrument (1), mit einem ersten Handhebel (2), der einen zum Betätigen per Hand vorgesehenen Handgriffabschnitt (3) an seinem proximalen Ende, einen Arbeitsabschnitt (4) zum Greifen, Klemmen oder Schneiden eines Objekts an seinem distalen Ende und einen dazwischenliegenden Lagerabschnitt (5) aufweist, an dem ein Lagerelement (6) angreift, um eine Schwenkbarkeit um eine Drehachse (7) zwischen dem ersten Handhebel (2) und einem zweiten Handhebel (8) zu ermöglichen, wobei der zweite Handhebel (8) einen Handgriffabschnitt (9) an seinem proximalen Ende, einen Arbeitsabschnitt (10) an seinem distalen Ende und einen dazwischenliegenden Führungsabschnitt (11) aufweist, an dem das Lagerelement (6) angreift, wobei der Führungsabschnitt (11) des zweiten Handhebels (8) auf einer Seite eine Einlegeausnehmung (12) besitzt, um den Lagerabschnitt (5) des ersten Handhebels (2) so aufzunehmen, dass zwei Führungsüberstände (13, 14) distal und proximal der Einlegeausnehmung (12) in einer Betriebsstellung, in der ein Greifen, Klemmen oder Schneiden stattfindet, den Lagerabschnitt (5) wenigstens abschnittsweise überdecken, wobei der Lagerabschnitt (5) des ersten Handhebels (2) durch ein für eine Beabstandung Kraft oder Widerstand zur Verfügung stellendes Abstandverursachungselement (23) so in der Betriebsstellung von dem Führungsabschnitt (11) des zweiten Handhebels (8) weggedrückt wird, dass sich ein Reinigungsspalt (27) zwischen dem ersten Handhebel (2) und dem zweiten Handhebel (8) ausbildet, **dadurch gekennzeichnet, dass** sich der Reinigungsspalt (27) über die gesamte Länge des Führungsabschnitts (11) erstreckt.

2. Medizinisches Instrument (1) nach Anspruch 1, **dadurch gekennzeichnet, dass** das Abstandverursachungselement (23) als ein separates oder integrales Bauteil ausgebildet ist.

3. Medizinisches Instrument (1) nach Anspruch 1 oder 2, **dadurch gekennzeichnet, dass** das Abstandverursachungselement (23) als ein Ring (25), ein Bund oder ein Steg ausgebildet ist.

4. Medizinisches Instrument (1) nach Anspruch 1 oder 2, **dadurch gekennzeichnet, dass** das Abstandverursachungselement (23) als eine Feder (26), ein Magnet oder ein Druckbehälter ausgebildet ist oder ein thermoaktives Element besitzt.

5. Medizinisches Instrument (1) nach einem der Ansprüche 1 bis 4, **dadurch gekennzeichnet, dass** das Abstandverursachungselement (23) derart ausgebildet ist, dass eine Reinigungsspalthöhe manuell einstellbar ist.

6. Medizinisches Instrument (1) nach einem der Ansprüche 1 bis 5, **dadurch gekennzeichnet, dass** das Lagerelement (6) als eine Schraube (24) oder ein Niet ausgebildet ist.

7. Medizinisches Instrument (1) nach Anspruch 1, 2 oder 4, **dadurch gekennzeichnet, dass** eine Reinigungsspalthöhe in der Betriebsstellung kleiner ist als die Reinigungsspalthöhe in einer Reinigungsstellung.

8. Medizinisches Instrument (1) nach Anspruch 7, **dadurch gekennzeichnet, dass** die Reinigungsstellung einer Montagestellung der beiden Handhebel (2, 8) entspricht.

9. Medizinisches Instrument (1) nach einem der Ansprüche 1 bis 8, **dadurch gekennzeichnet, dass** die relative Verschwenkbarkeit der beiden Handhebel (2, 8) zueinander durch einen an den Handgriffabschnitten (3, 9) vorgesehenen Rastmechanismus begrenzt ist.

10. Medizinisches Instrument (1) nach einem der Ansprüche 1 bis 9, **dadurch gekennzeichnet, dass** der Arbeitsabschnitt (4) des ersten Handhebels (2) und der Arbeitsabschnitt (10) des zweiten Handhebels (8) gleich oder unterschiedlich ausgebildet sind.

## Claims

1. A medical instrument (1) comprising a first hand lever (2) which has a manually actuated grip portion (3) at its proximal end, a work portion (4) for gripping, clamping or cutting an object at its distal end and, lying between these, a bearing portion (5) on which a bearing element (6) engages in order to permit pivotability about a rotation axis (7) between the first hand lever (2) and a second hand lever (8), wherein the second hand lever (8) has a grip portion (9) at its proximal end, a work portion (10) at its distal end and, lying between these, a guide portion (11) on which the bearing element (6) engages, wherein the guide portion (11) of the second hand lever (8) has on one side a recess (12) for receiving the bearing portion (5) of the first hand lever (2) such that two guide protrusions (13, 14) located distally and proximally of the recess (12) at least partially cover the bearing portion (5) in an operating position in which gripping, clamping or cutting takes place, wherein the bearing portion (5) of the first hand lever (2) is pressed, by means of a spacing element (23) that provides distance through force or resistance, away from the guide portion (11) of the second hand lever (8) in the operating position such that a cleaning gap (27) is formed between the first hand lever (2) and the second hand lever (8), **Characterized in that** the cleaning gap (27) extends along the entire length of the guide portion (11).

2. The medical instrument (1) according to claim 1, **characterized in that** the spacing element (23) is in the form of a separate or integral component.

3. The medical instrument (1) according to claim 1 or 2, **characterized in that** the spacing element (23) is in the form of a ring (25), a collar or a land.

4. The medical instrument (1) according to claim 1 or 2, **characterized in that** the spacing element (23) is in the form of a spring (26), a magnet or a pressure tank or has a thermally active element.

5. The medical instrument (1) according to any one of the claims 1 to 4, **characterized in that** the spacing element (23) is configured so that a cleaning gap height can be manually adjusted.

6. The medical instrument (1) according to any one of the claims 1 to 5, **characterized in that** the bearing element (6) is in the form of a screw (24) or a rivet.

7. The medical instrument (1) according to claim 1, 2 or 4, **characterized in that** a cleaning gap height is smaller in the operating position than the cleaning gap height in a cleaning position.

8. The medical instrument (1) according to claim 7, **characterized in that** the cleaning position corresponds to a mounting position of the two hand levers (2, 8).

9. The medical instrument (1) according to any one of the claims 1 to 8, **characterized in that** the pivotability of the two hand levers (2, 8) relative to each other is limited by a stop mechanism provided at the grip portions (3, 9).

10. The medical instrument (1) according to any one of the claims 1 to 9, **characterized in that** the work portion (4) of the first hand lever (2) and the work portion (10) of the second hand lever (8) are designed identically or differently.

## Revendications

1. Instrument médical (1), avec un premier levier à main (2) qui présente une section de poignée (3) prévue au niveau de son extrémité proximale pour actionner à la main, une section de travail (4) pour saisir, serrer ou couper un objet au niveau de son extrémité distale, et une section de palier (5) intermédiaire au niveau de laquelle un élément de palier (6) vient en prise pour permettre une mobilité de pivotement autour d'un axe de rotation (7) entre le premier levier à main (2) et un second levier à main (8), dans lequel le second levier à main (8) présente une section de poignée (9) au niveau de son extrémité proximale, une section de travail (10) au niveau de son extrémité distale et une section de guidage (11) intermédiaire au niveau de laquelle l'élément de palier (6) vient en prise, dans lequel la section de guidage (11) du second levier à main (8) possède sur un côté un évidement d'insertion (12) pour recevoir la section de palier (5) du premier levier à main (2) de telle sorte que deux saillies de guidage (13, 14) recouvrent au moins par endroits la section de palier (5), de façon distale et proximale par rapport à l'évidement d'insertion (12) et dans une position de fonctionnement dans laquelle une saisie, un serrage ou une découpe se produit, dans lequel la section de palier (5) du premier levier à main (2) est, dans la position de fonctionnement, repoussée de la section de guidage (11) du second levier à main (8), par un élément provoquant un espace (23) mettant à disposition une force ou une résistance pour un espacement, de telle sorte qu'une fente de nettoyage (27) se forme entre le premier levier à main (2) et le second levier à main (8), **caractérisé en ce que** la fente de nettoyage (27) s'étend sur toute la longueur de la section de guidage (11).

2. Instrument médical (1) selon la revendication 1, **caractérisé en ce que** l'élément provoquant un espace (23) est conçu en tant que composant séparé ou intégré.

3. Instrument médical (1) selon la revendication 1 ou 2, **caractérisé en ce que** l'élément provoquant un espace (23) est conçu en tant qu'anneau (25), épaulement ou traverse.

4. Instrument médical (1) selon la revendication 1 ou 2, **caractérisé en ce que** l'élément provoquant un espace (23) est conçu en tant que ressort (26), aimant ou réservoir sous pression ou possède un élément thermoactif.

5. Instrument médical (1) selon l'une des revendications 1 à 4, **caractérisé en ce que** l'élément provoquant un espace (23) est conçu de telle sorte qu'une hauteur de fente de nettoyage puisse être réglée manuellement.

6. Instrument médical (1) selon l'une des revendications 1 à 5, **caractérisé en ce que** l'élément de palier (6) est conçu en tant que vis (24) ou rivet.

7. Instrument médical (1) selon la revendication 1, 2 ou 4, **caractérisé en ce qu'**une hauteur de fente de nettoyage dans la position de fonctionnement est inférieure à la hauteur de fente de nettoyage dans une position de nettoyage.

8. Instrument médical (1) selon la revendication 7, **caractérisé en ce que** la position de nettoyage correspond à une position de montage des deux leviers à main (2, 8).

9. Instrument médical (1) selon l'une des revendications 1 à 8, **caractérisé en ce que** la mobilité de pivotement relative des deux leviers à main (2, 8) l'un par rapport à l'autre est limitée par un mécanisme d'encliquetage prévu au niveau des sections de poignée (3, 9).

10. Instrument médical (1) selon l'une des revendications 1 à 9, **caractérisé en ce que** la section de travail (4) du premier levier à main (2) et la section de travail (10) du second levier à main (8) sont conçues à l'identique ou différentes.
